Europäisches Patentamt

European Patent Office

Office européen des brevets

(19)

(11) EP 0 772 459 B1

(12) **EUROPEAN PATENT SPECIFICATION**

(45) Date of publication and mention
of the grant of the patent:
**19.03.2003 Bulletin 2003/12**

(21) Application number: **95918875.6**

(22) Date of filing: **01.05.1995**

(51) Int Cl.[7]: **A61K 51/08**

(86) International application number:
**PCT/US95/05340**

(87) International publication number:
**WO 95/029708 (09.11.1995 Gazette 1995/48)**

(54) **TECHNETIUM-99m LABELED IMAGING AGENTS**

TECHNETIUM-99M MARKIERTE BILDFORMUNGSMITTEL

AGENTS D'IMAGERIE MARQUES AU TECHNETIUM 99m

(84) Designated Contracting States:
**AT BE CH DE DK ES FR GB GR IE IT LI LU MC NL
PT SE**

(30) Priority: **02.05.1994 US 236402**

(43) Date of publication of application:
**14.05.1997 Bulletin 1997/20**

(73) Proprietor: **Diatide, Inc.
Londonderry, NH 03053 (US)**

(72) Inventors:
• **DEAN, Richard, T.
Bedford, NH 03110 (US)**
• **LISTER-JAMES, John
Bedford, NH 03110 (US)**
• **McBRIDE, William
Manchester, NH 03102 (US)**

(74) Representative: **Dörries, Hans Ulrich, Dr. et al
Dörries Frank-Molnia Pohlman
Postfach 22 16 61
80506 München (DE)**

(56) References cited:
**EP-A- 0 483 704       WO-A-90/15818
WO-A-92/13572       WO-A-93/10747
WO-A-93/12819       WO-A-93/23085
WO-A-94/00489       WO-A-94/23758
WO-A-94/26295       WO-A-95/00553
WO-A-95/03330**

• **THE JOURNAL OF NUCLEAR MEDICINE, vol. 35,
no. 5, May 1994 pages 257P-258P, J.
LISTER-JAMES ET AL. 'A
STRUCTURE-ACTIVITY-RELATIONSHIP (SAR)
STUDY OF SOMATOSTATIN
RECEPTOR-BINDING PEPTIDES
RADIOLABELED WITH TC-99M'**

**Description**

## BACKGROUND OF THE INVENTION

**Field of the Invention**

This invention relates to radiodiagnostic reagents and methods for producing labeled radiodiagnostic agents. Specifically, the invention relates to scintigraphic imaging agents for imaging sites in a mammalian body comprising specific-binding compounds labeled with technetium-99m (Tc-99m) *via* a radiolabel-binding moiety which forms a complex with Tc-99m. The invention provides radioactively-labeled scintigraphic imaging agents, reagents for preparing said radiolabeled scintigraphic imaging agents, methods for labeling said reagents and kits comprising non-radioactive embodiments of the reagents of the invention and other components for the convenient preparation of the scintigraphic imaging agents of the invention.

**Description of the Prior Art**

It is frequently clinically advantageous for a physician to be able to localize the site of pathological conditions in a patient using non-invasive means. Such pathological conditions include diseases of the lungs, heart, liver, kidneys, bones and brain, as well as cancer, thrombosis, pulmonary embolism, infection, inflammation and atherosclerosis.

In the field of nuclear medicine, certain pathological conditions are localized, or their extent is assessed, by detecting the distribution of small quantities of internally-administered radioactively labeled tracer compounds (called radiotracers or radiopharmaceuticals). Methods for detecting these radiopharmaceuticals are known generally as imaging or radioimaging methods.

In radioimaging, the radiolabel is a gamma-radiation emitting radionuclide and the radiotracer is located using a gamma-radiation detecting camera (this process is often referred to as gamma scintigraphy). The imaged site is detectable because the radiotracer is chosen either to localize at a pathological site (termed positive contrast) or, alternatively, the radiotracer is chosen specifically not to localize at such pathological sites (termed negative contrast). In many situations it is a particular advantage to use a radiolabeled specific binding compound as a radiopharmaceutical which localizes specifically to the pathological site *in vivo.*

A variety of radionuclides are known to be useful for radioimaging, including $^{67}$Ga, $^{99m}$Tc (Tc-99m), $^{111}$In, $^{123}$I, $^{125}$I, $^{169}$Yb or [186]Re. A number of factors must be considered for optimal radioimaging in humans. To maximize the efficiency of detection, a radionuclide that emits gamma energy in the 100 to 200 keV range is preferred. To minimize the absorbed radiation dose to the patient, the physical half-life of the radionuclide should be as short as the imaging procedure will allow. To allow for examinations to be performed on any day and at any time of the day, it is advantageous to have a source of the radionuclide always available at the clinical site. Tc-99m is a preferred radionuclide because it emits gamma radiation at 140 keV, it has a physical half-life of 6 hours, and it is readily available on-site using a molybdenum-99/technetium-99m generator. Other radionuclides used in the prior art are less advantageous than Tc-99m. This can be because the physical half-life of such radionuclides are longer, resulting in a greater amount of absorbed radiation dose to the patient (*e.g.,* indium-111). Alternatively, the gamma radiation energies of such alternate radionuclides are significantly lower (*e.g*., iodine-125) or higher (*e.g*., iodine-131) than Tc-99m and are thereby inappropriate for quality scintigraphic imaging. Lastly, many disadvantageous radionuclides cannot be produced using an on-site generator.

Tc-99m is a transition metal that is advantageously chelated by a metal complexing moiety. Radiolabel complexing moieties capable of binding Tc-99m can be covalently linked to various specific binding compounds to provide a means for radiolabeling such specific binding compounds. This is because the most commonly available chemical species of Tc-99m, pertechnetate ($TcO_4^-$), cannot bind directly to most specific binding compounds strongly enough to be useful as a radiopharmaceutical. Complexing of Tc-99m with such radiolabel complexing moieties typically entails chemical reduction of the pertechnetate using a reducing agent such as stannous chloride.

The use of chelating agents for complexing Tc-99m is known in the prior art.

Byrne *et al*., U.S. Patent No. 4,434,151 describe homocysteine thiolactone-derived bifunctional chelating agents that can couple radionuclides to terminal amino-containing compounds that are capable of localizing in an organ or tissue to be imaged.

Fritzberg, U.S. Patent No. 4,444,690 describes a series of technetium-chelating agents based on 2,3-bis (mercaptoacetamido) propanoate.

Byrne *et al*., U.S. Patent No. 4,571,430 describe novel homocysteine thiolactone bifunctional chelating agents for chelating radionuclides that can couple radionuclides to terminal amino-containing compounds that are capable of localizing in an organ or tissue to be imaged.

Byrne *et al*., U.S. Patent No. 4,575,556 describe novel homocysteine thiolactone bifunctional chelating agents for chelating radionuclides that can couple radionuclides to terminal amino-containing compounds that are capable of localizing in an organ or tissue to be imaged.

Nosco *et al.*, U.S. Patent No. 4,925,650 describe Tc-99m chelating complexes.

Kondo *et al.*, European Patent Application, Publication No. 483704 A1 disclose a process for preparing a Tc-99m complex with a mercapto-Gly-Gly-Gly moiety.

European Patent Application EP-A-0 135 160 describes bisamido, bisthiol Tc-99m ligands and salts thereof as renal function monitoring agents.

Davison *et al.*, 1981, *Inorg. Chem.* 20: 1629-1632 disclose oxotechnetium chelate complexes.

Fritzberg *et al.*, 1982, *J. Nucl. Med.* 23: 592-598 disclose a Tc-99m chelating agent based on N, N'-*bis*(mercaptoacetyl)-2,3-diaminopropanoate.

Byrne *et al.*, 1983, *J. Nucl. Med.* 24: P126 describe homocystine-containing Tc-99m chelating agents.

Bryson *et al.*, 1988, *Inorg. Chem.* 27: 2154-2161 describe neutral complexes of technetium-99 which are unstable to excess ligand.

Misra *et al.*, 1989, *Tet. Lett.* 30: 1885-1888 describe bisamine bisthiol compounds for radiolabeling purposes.

The use of chelating agents for radiolabeling specific-binding compounds is known in the art.

Gansow *et al.*, U.S. Patent No. 4,472,509 teach methods of manufacturing and purifying Tc-99m chelate-conjugated monoclonal antibodies.

Stavrianopoulos, U.S. Patent No. 4,943,523 teach detectable molecules comprising metal chelating moieties.

Fritzberg *et al.*, European Patent Application EP-A-0 188 256 describe dithiol, diamino, or diamidocarboxylic acid or amine complexes useful for making technetium-labeled imaging agents.

Albert *et al.*, UK Patent Application 8927255.3 disclose radioimaging using somatostatin derivatives such as octreotide labeled with $^{111}$In *via* a chelating group bound to the amino-terminus.

Albert *et al.*, International Patent Application No. WO 91/01144 disclose radioimaging using radiolabeled peptides related to growth factors, hormones, interferons and cytokines and comprised of a specific recognition peptide covalently linked to a radionuclide chelating group.

Fischman *et al.*, International Patent Application, Publication No. WO93/13317 disclose chemotactic peptides attached to chelating moieties.

Kwekkeboom *et al.*, 1991, J. Nucl. Med. 32: 981 Abstract #305 relates to radiolabeling somatostatin analogues with [111]In.

Albert *et al.*, 1991, Abstract LM10, 12th American Peptide Symposium: 1991 describe uses for $^{111}$In-labeled diethylene-triaminopentaacetic acid-derivatized somatostatin analogues.

Cox *et al.*, 1991, Abstract, 7th International Symposium on Radiopharmacology, p. 16, disclose the use of, Tc-99m-, $^{131}$I- and $^{111}$In-labeled somatostatin analogues in radiolocalization of endocrine tumors *in vivo* by scintigraphy.

Methods for labeling certain specific-binding compounds with Tc-99m are known in the prior art.

Hnatowich, U.S. Patent No. 4,668,503 describe Tc-99m protein raiolabeling.

Tolman, U.S. Patent No. 4,732,684 describe conjugation of targeting molecules and fragments of metallothionein.

Nicolotti *et al.*, U.S. Patent No. 4,861,869 describe bifunctional coupling agents useful in forming conjugates with biological molecules such as antibodies.

Fritzberg *et al.*, U.S. Patent No. 4,965,392 describe various S-protected mercaptoacetylglycylglycine-based chelators for labeling proteins.

Schochat *et al.*, U.S. Patent No. 5,061,641 disclose direct radiolabeling of proteins comprised of at least one "pendent" sulfhydryl group.

Fritzberg *et al.*, U.S. Patent No. 5,091,514 describe various S-protected mercaptoacetylglycylglycine-based chelators for labeling proteins.

Gustavson *et al.*, U.S. Patent No. 5,112,953 disclose Tc-99m chelating agents for radiolabeling proteins.

Kasina *et al.*, U.S. Patent No. 5,175,257 describe various combinations of targeting molecules and Tc-99m chelating groups.

Dean *et al.*, U.S. Patent No. 5,180,816 disclose methods for radiolabeling a protein with Tc-99m *via* a bifunctional chelating agent.

Sundrehagen, International Patent Application, Publication No. WO85/03231 disclose Tc-99m labeling of proteins. EP-A-0 237 150

Reno and Bottino, European Patent Application EP-A-0 237 150 disclose radiolabeling antibodies with Tc-99m.

Bremer *et al.*, European Patent Application EP-A-0 271 806 discloseTc-99m radiolabeling of antibody molecules.

Pak *et al.*, International Patent Application No. WO 88/07382 disclose a method for labeling antibodies with Tc-99m.

EP 0 772 459 B1

Goedemans *et al.*, PCT Application No. WO 89/07456 describe radiolabeling proteins using cyclic thiol compounds, particularly 2-iminothiolane and derivatives.

Dean *et al.*, International Patent Application, Publication No. WO89/12625 teach bifunctional coupling agents for Tc-99m labeling of proteins.

Schoemaker *et al.*, International Patent Application, Publication No. WO90/06323 disclose chimeric proteins comprising a metal-binding region.

Thornback *et al.*, EP-A-0 412 012 describe preparation and use of radiolabeled proteins or peptides using thiol-containing compounds, particularly 2-iminothiolane.

Gustavson *et al.*, International Patent Application, Publication No. WO91/09876 disclose Tc-99m chelating agents for radiolabeling proteins.

Rhodes, 1974, Sem. Nucl. Med. 4: 281-293 teach the labeling of human serum albumin with technetium-99m.

Khaw *et al.*, 1982, J. Nucl. Med. 23: 1011-1019 disclose methods for labeling biologically active macromolecules with Tc-99m.

Schwartz *et al.*, 1991, Bioconjugate Chem. 2: 333 describe a method for labeling proteins with Tc-99m using a hydrazinonicotinamide group.

Attempts at labeling peptides have been reported in the prior art.

Ege *et al.*, U.S. Patent No. 4,832,940 teach radiolabeled peptides for imaging localized T-lymphocytes.

Morgan *et al.*, U.S. Patent No. 4,986,979 disclose methods for imaging sites of inflammation.

Flanagan *et al.*, U.S. Patent No. 5,248,764 describe conjugates between a radiolabel chelating moiety and atrial natiuretic factor-derived peptides.

Ranby *et al.*, 1988, WO 89/00051 disclose a method for detecting fibrin deposits in an animal comprising covalently binding a radiolabeled compound to fibrin.

Lees *et al.*, 1989, WO 89/10760 teach radiolabeled peptides for arterial imaging.

Morgan *et al.*, International Patent Application, Publication No. WO90/10463 disclose methods for imaging sites of inflammation.

Flanagan *et al.*, European Patent Application EP-A-0 403 243 disclose Tc-99m labeling of synthetic peptide fragments via a set of organic chelating molecules.

Stuttle, PCT Application, Publication No. WO 90/15818 describes Tc-99m labeling of RGD-containing oligopeptides.

Rodwe *et al.*, 1991, WO 91/17173 disclose conjugates of "molecular recognition units" with "effector domains".

Cox, International Patent Application WO 92/21383 discloses radiolabeled somatostatin derivatives containing two cysteine residues.

Rhodes *et al.,* International Patent Application, Publication No. WO93/12819 teach peptides comprising metal ion-binding domains.

Lyle *et al*, International Patent Application, Publication No. WO93/15770 disclose Tc-99m chelators and peptides labeled with Tc-99m.

Coughlin *et al*, International Patent Application, Publication No. WO93/211 disclose bifunctional chelating agents comprising thiourea groups for radiolabelingtargeting molecules.

Knight *et al.*, 1990, 37th Annual Meeting of the Society of Nuclear Medicine, Abstract #209, claim thrombus imaging using Tc-99m labeled peptides.

Babich *et al.,* 1993, *J. Nucl. Med.* 34: 1964-1974 describe Tc-99m labeled peptides comprising hydrazinonicotinamide derivatives.

The use of chelating agents for radiolabeling peptides, and methods for labeling peptides with Tc-99m are known in the prior art and are disclosed in co-pending U.S. Patent Applications Serial Nos. 07/653,012, 07/807,062, 07/871,282, 07/886,752, 07/893,981, 07/955,466, 08/019,864, and 08/073,577, and radiolabeled peptides for use as scintigraphic imaging agents for imaging thrombi are known in the prior art and are disclosed in co-pending U.S. Patent Applications Serial Nos. 07/886,752,07/893,981 and 08/044,825 which are hereby incorporated by reference.

## SUMMARY OF THE INVENTION

The present invention provides reagents useful in preparing radiolabeled scintigraphic imaging agents. Specifically, the invention provides reagents for preparing scintigraphic imaging agents that are radiolabeled with technetium-99m (Tc-99m). The reagents of the invention are each comprised of a specific binding compound, including but not limited to peptides, that binds specifically and with high affinity to a site of interest in a mammalian body, and that is covalently linked to a radiolabel-complexing moiety.

In preferred embodiments, the invention provides reagents wherein the specific binding compounds are linear or cyclic peptides having an amino acid sequence of 4 to 100 amino acids.

Small compounds, preferably having a molecular weight of less than 10,000 daltons, are of distinct commercial

4

advantage. Such small compounds can be readily manufactured. Moreover, they are likely not to be immunogenic and to clear rapidly from the vasculature, thus allowing for better and more rapid imaging. In contrast, larger molecules such as antibodies or fragments thereof, or other biologically-derived peptides larger than 10,000 daltons, are costly to manufacture, and are likely to be immunogenic and clear more slowly from the bloodstream, thereby interfering with rapid diagnoses *in vivo.*

One aspect of the invention provides a reagent for preparing a radiolabeled scintigraphic imaging agent for imaging a site within a mammalian body, comprising a specific binding compound that specifically binds to the site in the mammalian body, and that is covalently linked to a Tc-99m complexing moiety of formula:

I.

$$R^1\text{-CO-(amino acid)}^1\text{-(amino acid)}^2\text{-Z}$$

wherein (amino acid)$^1$ and (amino acid)$^2$ are each independently any primary $\alpha$- or $\beta$-amino acid that does not comprise a thiol group, Z is a thiol-containing moiety that is cysteine, homocysteine, isocysteine, penicillamine, 2-mercaptoethylamine or 3-mercaptopropylamine, and R$^1$ is lower (C$^1$-C$^4$) alkyl, an amino acid or a peptide comprising 2 to 10 amino acids. When Z is cysteine, homocysteine, isocysteine or penicillamine, the carbonyl group of said moiety is covalently linked to a hydroxyl group, a NR$^3$R$^4$ group, wherein each of R$^3$ and R$^4$ are independently H or lower (C$^1$-C$^4$) alkyl, an amino acid or a peptide comprising 2 to 10 amino acids; or

II.

$$Y\text{-(amino acid)}^2\text{-(amino acid)}^1\text{-NHR}^2$$

wherein Y is a thiol-containing moiety that is cysteine, homocysteine, isocysteine, penicillamine, 2-mercaptoacetate or 3-mercaptopropionate, (amino acid)$^1$ and (amino acid)$^2$ are each independently any primary $\alpha$- or $\beta$-amino acid that does not comprise a thiol group, and R$^2$ is H or lower (C$^1$-C$^4$) alkyl, an amino acid or a peptide comprising 2 to 10 amino acids. When Y is cysteine, homocysteine, isocysteine or penicillamine, the amino group of said moiety is covalently linked to -H, an amino acid or a peptide comprising 2 to 10 amino acids.

The Tc-99m complexing moieties of the invention are covalently linked to the specific binding compound of each of the reagents of the invention through R$^1$, R$^2$, a sidechain group of the sidechain of (amino acid)$^1$ or (amino acid)$^2$, or the amino or carboxyl group of cysteine, homocysteine, isocysteine or penicillamine.

In one embodiment of the reagents of the invention, the radiolabel-complexing moiety has a formula that is -(amino acid)$^1$-(amino acid)$^2$-(amino thiol) or (mercaptocarboxylic acid)-(amino acid)$^1$-(amino acid)$^2$-, wherein (amino acid)$^1$ and (amino acid)$^2$ are each independently any naturally-ocurring, modified, substituted or altered primary $\alpha$- or $\beta$-amino acid; (amino thiol) is selected fromn the group consisting of cysteine, isocysteine, homocysteine, penicilamine, 2-mercaptoethylamine, and 3-mercaptopropylamine; and (mercaptocarboxylic acid) is selected fromn the group consisting of cysteine, isocysteine, homocysteine, penicilamine, 2-mercaptoacetic acid, and 3-mercaptoproprionic acid.

In preferred embodiments, the Tc-99m complexing moiety of the invention comprise moieties having the formulae -Gly-Gly-Cys- or Cys-Gly-Gly-.

The reagents of the invention may be formed wherein the specific binding compounds or the radiolabel-complexing moieties are covalently linked to a polyvalent linking moiety. Polyvalent linking moieties of the invention are comprised of at least 2 identical linker functional groups capable of covalently bonding to specific binding compounds or radiolabel- complexing moieties. Preferred linker functional groups are primary or secondary amines, hydroxyl groups, carboxylic acid groups or thiol-reactive groups. In preferred embodiments, the polyvalent linking moieties are comprised of *bis*-succinimdylmethylether (BSME), 4-(2,2-dimethylacetyl)benzoic acid (DMAB), *tris*(succinimidylethyl) amine (TSEA), 4-(O-CH$_2$CO-Gly-Gly-Cys.amide)acetophenone (ETAC), *bis*-succinimidohexane (BSH), *tris*(2-chloroacetamido-ethyl)amine, and 1,2-*bis*-[2-(chloroacetamido)ethoxy]ethane.

The invention also comprises scintigraphic imaging agents that are complexes of the reagents of the invention with Tc-99m and methods for radiolabeling the reagents. Tc-99m radiolabeled complexes provided by the invention are formed by reacting the reagents of the invention with Tc-99m in the presence of a reducing agent. Preferred reducing agents include but are not limited to dithionite ion, stannous ion and ferrous ion. Complexes of the invention are also formed by labeling the reagents of the invention with Tc-99m by ligand exchange of a prereduced Tc-99m complex as provided herein.

The invention also provides kits for preparing scintigraphic imaging agents that are the reagents of the invention radiolabeled with Tc-99m. Kits for labeling the reagents provided by the invention with Tc-99m are comprised of a sealed vial containing a predetermined quantity of a reagent of the invention and a sufficient amount of reducing agent to label the reagent with Tc-99m.

This invention provides methods for preparing peptide embodiments of the reagents of the invention by chemical synthesis *in vitro*. In a preferred embodiment, peptides are synthesized by solid phase peptide synthesis.

This invention provides methods for using scintigraphic imaging agents that are Tc-99m labeled reagents for imaging sites within a mammalian body by obtaining *in vivo* gamma scintigraphic images. These methods comprise administering an effective diagnostic amount of Tc-99m labeled reagents of the invention and detecting the gamma radiation emitted by the Tc-99m label localized at the site within the mammalian body.

Specific preferred embodiments of the present invention will become evident from the following more detailed description of certain preferred embodiments and the claims.

## BRIEF DESCRIPTION OF THE DRAWINGS

Figure 1 shows the deposition pattern of Sudan IV in 4 HC-treated and 1 control rabbit aortae.

Figure 2 shows *ex corpora* images of radiotracer uptake and plaque visualization in 4 HC-treated and 1 control rabbit aortae.

Figure 3 shows the *in vivo* image in an animal of radiotracer uptake and plaque localization in the aortic arch.

Figure 4 shows an image of $^{99m}$Tc-labeled P587 in a tumor-bearing rat, indicated by an arrow, showing high uptake at the tumor site in the lower leg.

## DETAILED DESCRIPTION OF PREFERRED EMBODIMENTS

The present invention provides reagents, including peptide reagents, for preparing Tc-99m labeled scintigraphic imaging agents for imaging target sites within a mammalian body. The reagents provided by the invention comprise a radiolabel complexing moiety covalently linked to a specific binding compound that binds to a site within a mammalian body, radiolabeled with Tc-99m.

Labeling with Tc-99m is an advantage of the present invention because the nuclear and radioactive properties of this isotope make it an ideal scintigraphic imaging agent. This isotope has a single photon energy of 140 keV and a radioactive half-life of about 6 hours, and is readily available from a $^{99}$Mo-$^{99m}$Tc generator. Another advantage of the present invention is that none of the preferred radionuclides are toxic, in contrast to other radionuclides known in the art (*for example*, $^{125}$I).

For purposes of this invention, the term "specific binding compound" is intended to mean any compound that specifically binds to a target site in a mammalian body. "Specific binding" will be understood by those with skill in this art as meaning that the compound localizes to a greater extent at the target site than to surrounding tissues. Such specific binding is advantageous because scintigraphic imaging agents comprising such specific binding compounds are distributed within a mammalian body after administration to provide visual definition of the target *in vivo.* Specific binding compounds include but are not limited to peptide, oligosaccharides, nucleotides, oligonucleotides and polynucleotides, and specific receptor-binding compounds.

Each specific-binding peptide-containing embodiment of the invention is comprised of a sequence of amino acids. The term amino acid as used in this invention is intended to include all ∟- and ᴅ-, primary α- and β-amino acids, naturally occurring, modified, substituted, altered and otherwise. Specific binding peptide embodiments of the reagents of the invention comprise specific binding peptides having a molecular weight of about 5,000 daltons. Particularly preferred embodiments of the specific binding peptides of the invention include peptides that bind with high affinity to the platelet GPIIb/HIa receptor. In additional preferred embodiments, the specific binding peptides include peptides that bind specifically to the somatostatin receptor (SSTR) on SSTR-expresing cells, particularly tumor cells and activated T-lymphocyte cells. Reagents comprising specific-binding peptides provided by the invention include but are not limited to reagents comprising peptides having the following amino acid sequences (the amino acids in the following peptides are L-amino acids except where otherwise indicated):

cyclo(*N*-methyl)FYW_DKV.Hcy.(CH$_2$CO.GGC.amide)
cyclo(*N*-methyl)FYW_DKV.Hcy.(CH$_2$CO.GGCK.amide)
cyclo(*N*-methyl)FYW_DKV.Hcy.(CH$_2$CO.GGCR.amide)
cyclo(*N*-methyl)FYW_DKV.Hcy.(CH$_2$CO.GGCRD.amide)
cyclo(*N*-methyl)FYW_DKV.Hcy.(CH$_2$CO.GGCRK.amide)
cyclo(*N*-methyl)FYW_DKV.Hcy.(CH$_2$CO.GGCRR.amide)
cyclo(*N*-methyl)FYW_DKV.Hcy.(CH$_2$CO.GGCKK.amide)

*cyclo*(*N*-methyl)FYW$_D$KV.Hcy.(CH$_2$CO.GGCKKK.amide)
*cyclo*(*N*-methyl)FYW$_D$KV.Hcy.(CH$_2$CO.GGC.Orn.amide)
*cyclo*(*N*-methyl)FYW$_D$KV.Hcy.(CH$_2$CO.GGCKDK.amide)
*cyclo*(*N*-methyl)FYW$_D$KV.Hcy.(CH$_2$CO.GGC.Orn.D.Orn.amide)
*cyclo*(*N*-methyl)FYW$_D$KV.Hcy.(CH$_2$CO.GGC.Orn.D.amide)
*cyclo*(*N*-methyl)FYW$_D$KV.Hcy.(CH$_2$CO.KKC.amide)
*cyclo*(*N*-methyl)FYW$_D$KV.Hcy.(CH$_2$CO.KRC.amide)
*cyclo*(*N*-methyl)FYW$_D$KV.Hcy.(CH$_2$CO.RRC.amide)
*cyclo*(*N*-methyl)FYW$_D$KV.Hcy.(CH$_2$CO.KKCK.amide)
*cyclo*(*N*-methyl)FYW$_D$KV.Hcy.(CH$_2$CO.GRCK.amide)
*cyclo*(*N*-methyl)FYW$_D$KV.Hcy.(CH$_2$CO.GKCR.amide)
CH$_2$CO.Y$_D$.Apc.GDCGGC$_{Acm}$GC$_{Acm}$GGC.amide
CH$_2$CO.Y$_D$.Apc.GDCGGC$_{Acm}$GC$_{Acm}$GGCG.amide
CH$_2$CO.Y$_D$.Apc.GDCGGSSGGCG.amide
CH$_2$CO.Y$_D$.Apc.GDCGGCG.amide
CH$_2$CO.Y$_D$.Amp.GDCKGCG.amide

(Single-letter abbreviations for amino acids can be found in G. Zubay, *Biochemistry (2d.* ed.), 1988 (MacMillen Publishing: New York) p.33; other abbreviations are as in the Legend to Table I). This list of reagents provided by the invention is illustrative and not intended to be limiting or exclusive, and it will be understood by those with skill in the art that reagents comprising combinations of the peptides disclosed herein or their equivalents may be covalently linked to any of the chelating moieties of the invention and be within its scope, including combinations of such peptides and chelating moieties comprising linking groups as disclosed herein.

For optimal imaging using one embodiment of the invention, the reagent must be capable of binding to the platelet GPIIb/IIIa receptor with sufficient affinity that it inhibits the adenosine diphosphate (ADP)-induced aggregation of human platelets in a standard platelet aggregation assay *(see* Example 3 *below)* when present at a concentration of no more than 0.3 μM.

In certain embodiments of the reagents of the invention, β-glucans comprise the specific binding compound component. For the purposes of this invention, the term "β-glucan" is intended to mean oligosaccharides comprising 1,3- and 1,6-linked β-$_D$-glucose residues wherein the β-glucan moiety has a molecular weight of up to about 2,000 kilodaltons. One preferred embodiment of β-glucan-containing reagents of the invention has formula:

β-glucan-(=NNHCO.(CH$_2$)$_3$CO.)GGC.amide

Polyvalent linking moieties are covalently linked to the specific peptides of the invention, the Tc-99m complexing moieties, or both. Polyvalent linking moieties provided by the invention are comprised of at least 2 linker functional groups capable of covalently bonding to specific binding peptides or Tc-99m complexing moieties. Such functional groups include but are not limited to primary and secondary amines, hydroxyl groups, carboxylic acid groups and thiol reactive groups. Polyvalent linking moieties are comprised of preferably at least three functional groups capable of being covalently linked to specific binding peptides or technetium-99m complexing moieties. Preferred polyvalent linking moieties include amino acids such as lysine, homolysine, ornithine, aspartic acid and glutamic acid; linear and cyclic amines and polyamines; polycarboxylic acids; and activated thiols such as di- and tri-maleimides. Also preferred are embodiments wherein the polyvalent linking moieties comprise a multiplicity of polyvalent linking moieties covalently linked to form a branched polyvalent linking moiety.

Specific-binding peptides comprising the reagents of the present invention can be chemically synthesized *in vitro.* Such peptides can generally advantageously be prepared on an amino acid synthesizer. The peptides of this invention can be synthesized wherein the radiolabel-binding moiety is covalently linked to the peptide during chemical synthesis *in vitro*, using techniques well known to those with skill in the art. Such peptides covalently-linked to the radiolabel-binding moiety during synthesis are advantageous because specific sites of covalent linkage can be determined.

Radiolabel complexing moieties of the invention may be introduced into the target specific peptide during peptide synthesis. The radiolabel complexing moiety can be introduced into the peptide to comprise the amino- or carboxyl-terminus of the peptide. In addition, radiolabel-complexing moieties may be covalently linked to the groups comprising the sidechains of amino acids, *for example,* the ∈-amino group of lysine to give, for example, αN(Fmoc)-Lys-∈N[Gly-Gly-Cys], which may be incorporated at any position in the peptide chain. This sequence is particularly advantageous as it affords an easy mode of incorporation into the target binding peptide. This invention provides for the incorporation of these chelators into virtually any peptide, resulting in a radiolabeled peptide covalently linked to a Tc-99m complexing moiety.

In forming a complex of radioactive technetium with the reagents of this invention, the technetium complex, preferably a salt of Tc-99m pertechnetate, is reacted with the reagents of this invention in the presence of a reducing agent. Preferred reducing agents are dithionite, stannous and ferrous ions; the most preferred reducing agent is stannous chloride. In an additional preferred embodiment, the reducing agent is a solid-phase reducing agent. Complexes and means for preparing such complexes are conveniently provided in a kit form comprising a sealed vial containing a predetermined quantity of a reagent of the invention to be labeled and a sufficient amount of reducing agent to label the reagent with Tc-99m. Alternatively, the complex may be formed by reacting a reagent of this invention with a pre-formed labile complex of technetium and another compound known as a transfer ligand. This process is known as ligand exchange and is well known to those skilled in the art. The labile complex may be formed using such transfer ligands as tartrate, citrate, gluconate or mannitol, for example. Among the Tc-99m pertechnetate salts useful with the present invention are included the alkali metal salts such as the sodium salt, or ammonium salts or lower alkyl ammonium salts.

In a preferred embodiment of the invention, a kit for preparing technetium-99m labeled reagents is provided. An appropriate amount of a reagent is introduced into a vial containing a reducing agent, such as stannous chloride or a solid-phase reducing agent, in an amount sufficient to label the reagent with Tc-99m. An appropriate amount of a transfer ligand as described (such as tartrate, citrate, gluconate or mannitol, for example) can also be included. Technetium-99m labeled scintigraphic imaging agents according to the present invention can be prepared by the addition of an appropriate amount of Tc-99m or Tc-99m complex into the vials and reaction under conditions described in Example 2 hereinbelow. The kit may also contain conventional pharmaceutical adjunct materials such as, for example, pharmaceutically acceptable salts to adjust the osmotic pressure, buffers, preservatives and the like. The components of the kit may be in liquid, frozen or dry form. In a preferred embodiment, kit components are provided in lyophilized form. Radiolabeled scintigraphic imaging reagents according to the present invention may be prepared by reaction under conditions described in Example 2 hereinbelow.

Radioactively labeled reagents provided by the present invention are provided having a suitable amount of radioactivity. In forming Tc-99m radioactive complexes, it is generally preferred to form radioactive complexes in solutions containing radioactivity at concentrations of from about 0.01 millicurie (mCi) to 100 mCi per mL.

Technetium-99m labeled scintigraphgic imaging agents provided by the present invention can be used for visualizing sites in a mammalian body. In accordance with this invention, the technetium-99m labeled scintigraphic imaging agents are administered in a single unit injectable dose. Any of the common carriers known to those with skill in the art, such as sterile saline solution or plasma, can be utilized after radiolabeling for preparing the injectable solution to diagnostically image various organs, tumors and the like in accordance with this invention. Generally, the unit dose to be administered has a radioactivity of about 0.01 mCi to about 100 mCi, preferably 1 mCi to 20 mCi. The solution to be injected at unit dosage is from about 0.01 mL to about 10 mL. After intravenous administration, imaging of the organ or tumor *in vivo* can take place in a matter of a few minutes. However, imaging can take place, if desired, in hours or even longer, after the radiolabeled reagent is injected into a patient. In most instances, a sufficient amount of the administered dose will accumulate in the area to be imaged within about 0.1 of an hour to permit the taking of scintiphotos. Any conventional method of scintigraphic imaging for diagnostic purposes can be utilized in accordance with this invention.

The technetium-99m labeled reagents and complexes provided by the invention may be administered intravenously in any conventional medium for intravenous injection such as an aqueous saline medium, or in blood plasma medium. Such medium may also contain conventional pharmaceutical adjunct materials such as, for example, pharmaceutically acceptable salts to adjust the osmotic pressure, buffers, preservatives and the like. Among the preferred media are normal saline and plasma.

The methods for making and labeling these compounds are more fully illustrated in the following Examples. These Examples illustrate certain aspects of the above-described method and advantageous results. These Examples are shown by way of illustration and not by way of limitation.

### EXAMPLE 1

### Solid Phase Peptide Synthesis

Solid phase peptide synthesis (SPPS) was carried out on a 0.25 millimole (mmole) scale using an Applied Biosystems Model 431A Peptide Synthesizer and using 9-fluorenylmethyloxycarbonyl (Fmoc) amino-terminus protection, coupling with dicyclohexylcarbodiimide/hydroxybenzotriazoleor2-(1H-benzo-triazol-1-yl)-1,1,3,3-tetramethyluronium hexafluorophosphate/ hydroxybenzotriazole (HBTU/HOBT), and using *p*-hydroxymethylphenoxy-methylpolystyrene (HMP) or Sasrin™ resin for carboxyl-terminus acids or Rink amide resin for carboxyl-terminus amides.

Homocysteine (Hcy) was prepared by alkaline hydrolysis oh L-homocysteine lactone. Fmoc.Hcy(*S*-trityl) and Fmoc.Pen(*S*-trityl) were prepared from the appropriate by tritylation with triphenylmethanol in trifluoroacetic acid, followed by Fmoc derivitization as described by Atherton *et al*. (1989, Solid Phase Peptide Synthesis, IRL Press: Oxford). 4-piperidinyl butyl ether derivatives of tyrosine (Y[(CH$_2$)$_4$-piperidine]) were prepared by SPPS starting with Fmoc-tyrosine-(4-Boc-piperidine butyl ether). Fmoc-*S*-(3-Boc-aminopropyl)cysteine was prepared in L-cysteine and Boc-aminopropyl bromide in methanolic sodium methoxide followed by treatment with O-9-fluorenylmethyl-O'-*N*-succcinimidyl carbonate (FmocOSu) at pH 10.

Where appropriate, 2-haloacetyl groups were introduced either by using the appropriate 2-haloacetic acid as the last residue to be coupled during SPPS or by treating the N-terminus free amino peptide bound to the resin with either the 2-haloacetic acid/ diisopropylcarbodiimide/*N*-hydroxysuccinimide in NMP of the 2-halo-acetic anhydride/ diisopropylethylamine in NMP.

Where appropriate, 2-haloacetylated peptides were cyclized by stirring an 0.1 - 1.0 mg/mL solution in phosphate or bicarbonate buffer or dilute ammonium hydroxide (pH 8) containing 0.5 - 1.0 mM EDTA for 4 - 48 hours, followed by acidification with acetic acid, lyophilization and HPLC purification.

Where appropriate, thiol-containing peptides were reacted with chloroacetyl-containing, thiol-protected Tc-99m complexing moieties at pH 10 for 0.5-4 hours at room temperature, followed by acetic acid acidification and evaporation of the solution to give the corresponding peptide-sulfide adduct. Deprotection and purification were routinely performed as described to yield the chelator-peptide conjugate.

Sasrin™ resin-bound peptides were cleaved using a solution of 1% TFA in dichloromethane to yield the protected peptide. Where appropriate, protected peptidde precursors were cyclized between the amino- and carboxyl-termini by reaction of sidechain-protected, amino-terminal free amine and carboxyl-terminal free acid using diphenylphosphorylazide.

HMP or Rink amide resin-bound products were routinely cleaved and protected cyclized peptides deprotected using a solution comprised of trifluoroacetic acid (TFA), or TFA and methylene chloride, optionally comprising water, thioanisole, ethanedithiol, and triethylsilane in ratios of 100 : 5 : 5 : 2.5 : 2, for 0.5 - 3 hours at room temperature. Where appropriate, products were re-*S*-tritylated in triphenolmethanol/TFA, and *N*-Boc groups re-introduced into the peptide using (Boc)$_2$O.

Crude peptides were purified by preparative high pressure liquid chromatography (HPLC) using a Waters Delta-Pak C18 column and gradient elution with 0.1% TFA in water modified with acetonitrile. After column elution, acetonitrile was evaporated from the eluted fractions, which were then lyophilized. The identity of each product so produced andd purified was confirmed by fast atom bombardment mass spectroscopy (FABMS) or electrospray mass spectroscopy (ESMS).

## EXAMPLE 2

### A General Method for Radiolabeling with Tc-99m

0.1 mg of a peptide reagent prepared as in Example 1 was dissolved in 0.1 mL of water, or 50:50 ethanol: water, or phosphate-buffered saline (PBS), or 50mM potassium phosphate buffer (pH = 5, 6 or 7.4). Tc-99m gluceptate was prepared by reconstituting a Glucoscan vial (E.I. DuPont de Nemours, Inc.) with 1.0 mL of Tc-99m sodium pertechnetate containing up to 200 mCi and allowed to stand for 15 minutes at room temperature. 25 μl of Tc-99m gluceptate was then added to the reagent and the reaction allowed to proceed at room temperature or at 100°C for 15-30 min and then filtered through a 0.2 μm filter.

The Tc-99m labeled peptide reagent purity was determined by HPLC using the following conditions: a Waters Delta-Pak RP-18 analytical column, having dimensions of 5μm x 4.6mm x 220mm, was loaded with each radiolabeled peptide, which were then eluted at a solvent flow rate of 1mL/min. Gradient elution was performed over 10-20 min using a linear gradient beginning with 100% Solvent A (0.1% TFA/water) and ending with 100% Solution B (0.1% TFA/ 90% acetonitrile/water). Radioactive components were detected by an in-line radiometric detector linked to an integrating recorder. Tc-99m gluceptate and Tc-99m sodium pertechnetate elute between 1 and 4 minutes under these conditions, whereas the Tc-99m labeled peptide eluted after a much greater amount of time.

The following Table illustrates successful Tc-99m labeling of peptides prepared according to Example 1 using the method described herein.

## TABLE I

| Peptides | FABMS MH$^+$ | Radiochemical Yield(%)[*] | HPLC R$_T$(min)[**] |
|---|---|---|---|
| *cyclo*(N-methyl)FYW$_D$KV.Hcy.(cH₂co.GGC.amide) | 1129 | 98[4] | 15.1, 17.2 |
| *cyclo*(N-methyl)FYW$_D$KV.Hcy.(cH₂co.GGCK.amide) | 1258 | 99[4] | 15.0 |
| *cyclo*(N-methyl)FYW$_D$KV.Hcy.(cH₂co.GGCR.amide) | 1285 | 99[3] | 15.1 |
| *cyclo*(N-methyl)FYW$_D$KV.Hcy.(cH₂co.GGCKK.amide) | 1386 | N.D. | N.D. |
| *cyclo*(N-methyl)FYW$_D$KV.Hcy.(cH₂co.GGC.Orn.amide) | 1244 | 98[6] | 7.0[1] |
| cH₂co.Y$_D$.Apc.GDCGGC$_{Acm}$GC$_{Acm}$GGC.amide | 1393 | 97[4] | 11.4[3] |
| cH₂co.Y$_D$.Apc.GDCGGSSGGCG.amide | 1219 | N.D. | N.D. |
| cH₂co.Y$_D$.Apc.GDCGGCG.amide | 930 | 99 | 11.3[3] |
| cH₂co.Y$_D$.Apc.GDCGGC$_{Acm}$GC$_{Acm}$GGCG.amide | 1450[7] | N.D. | N.D. |

EP 0 772 459 B1

* Superscripts refer to the following labeling conditions:

[1] = in water at room temperature
[2] = in 0.05M phosphate, pH 7.4, at room temperature
[3] = in 10% HPCD at room temperature
[4] = in 50/50 ethanol/water at room temperature
[5] = in pH 9 at 100°C
[6] = in 0.9% NaCl at 100°C

** HPLC methods (indicated by superscript after $R_T$):

1 = Waters-1 column, 100% Solution A → 100% Solution B in 10 min
2 = Vydac column, 100% Solution A → 100% Solution B in 10 min
3 = Waters-2 column, 100% Solution A → 100% Solution B in 20 min

Single-letter abbreviations for amino acids can be found in G. Zubay, *Biochemistry* (2d. ed.), 1988 (MacMillen Publishing: New York) p.33. Underlining indicates the formation of an amide or a thiol linkage between the linked amino acids of derivative groups. Acm is acetamidomethyl; Orn is ornithine; $F_D$ is $_D$-phenylalanine; $Y_D$ is $_D$-tyrosine; $W_D$ is $_D$-tryptophan; Apc = $_L$-[S-(3-aminopropyl)cysteine; Amp is 4-amidino-phenylalanine; and Hcy is homocysteine.

## EXAMPLE 3

**Platelet Aggregation Inhibition Assays**

Platelet aggregation studies were performed essentially as described by Zucker (1989, Methods in Enzymol. 169: 117-133). Briefly, platelet aggregation was assayed with or without putative platelet aggregation inhibitory compounds using fresh human platelet-rich plasma, comprising 300,000 platelets per microlitre. Platelet aggregation was induced by the addition of a solution of adenosine diphosphate to a final concentration of 10 to 15 micromolar, and the extent of platelet aggregation monitored using a Bio/Data aggregometer (Bio/Data Corp., Horsham, PA). The concentrations of platelet aggregation inhibitory compounds used were varied from 0.1 to 500 µg/mL. The concentration of inhibitor that reduced the extent of platelet aggregation by 50% (defined as the $IC_{50}$) was determined from plots of inhibitor concentration *versus* extent of platelet aggregation. An inhibition curve for peptide RGDS was determined for each batch of platelets tested as a positive control.

The results of these experiments are shown in Table II. In Table II, the compounds tested are as follows (RGDS is given as a positive control):

## TABLE II

| | | | |
|---|---|---|---|
| P686 | = | $CH_3CO.Y_D.Apc.\underline{GDC}GGSSGGCG.amide$ | $IC_{50} = 0.34\mu M$ |
| P246 | = | $CH_3CO.Y_D.Apc.\underline{GDC}GGC_{Acm}GC_{Acm}GGC.amide$ | $IC_{50} = 0.63\mu M$ |
| P645 | = | $CH_3CO.Y_D.Amp.\underline{GDC}GGC_{Acm}GC_{Acm}GGC.amide$ | $IC_{50} = 0.67\mu M$ |
| P665 | = | $CH_3CO.Y_D.Apc.\underline{GDC}GGSSGGCG.amide$ | $IC_{50} = 0.80\mu M$ |
| P676 | = | $CH_3CO.Y_D.Apc.\underline{GDC}GGC_{Acm}GC_{Acm}GGCG.amide$ | $IC_{50} = 0.97\mu M$ |

(Single-letter abbreviations for amino acids can be found in G. Zubay, *Biochemistry* (2d. ed.), 1988 (MacMillen Publishing: New York) p.33 as discussed in the Legend of Table I; Acm = acetamidomethyl; Amp = 4-amidinophenylalanine; Apc = $_L$-[S-(3-aminopropyl)cysteine; $Y_D$ = $_D$-tyrosine.

These results demonstrate that peptide reagents of the inveniton bind with high affinity to specific GPIIb/IIIa receptors *in vitro.*

## EXAMPLE 4

### *In Vivo* Imaging of Deep Vein Thrombosis using a Tc-99m Labeled Peptide in a Canine Model

Mongrel dogs (25-35lb., fasted overnight) are sedated with a combination of ketamine and aceprozamine intramuscularly and then anesthetized with sodium pentabarbital intravenously. In each animal, an 18-gauge angiocath is inserted in the distal half of the right femoral vein and an 8mm Dacron®-entwined stainless steel embolization coil (Cook Co., Bloomington IN) is placed in the femoral vein at approximately mid-femur. The catheter is removed, the wound sutured and the placement of the coil documented by X-ray. The animals are then allowed to recover overnight.

One day following coil placement, each animal is re-anesthetized, intravenous saline drips placed in each foreleg and a urinary bladder catheter inserted to collect urine. The animal is placed supine under a gamma camera equipped with a low-energy, all purpose collimator and photopeaked for Tc-99m. Tc-99m labeled peptide [185-370 mBq (5-10 mCi) Tc-99m, 0.2-0.4 mg reagent] is injected sequentially into one foreleg intravenous line at its point of insertion. The second line is maintained for blood collection.

Gamma camera imaging is started simultaneously with injection. Anterior images over the heart are acquired as a dynamic study (10 sec image acquisitions) over the first 10 min, and then as static images at 1, 2, 3 and 4h post-injection. Anterior images over the legs are acquired for 500,000 counts or 20 min (whichever is shorter), at approximately 10-20 min, and at approximately 1, 2, 3 and 4h post-injection. Leg images are collected with a lead shield placed over the bladder.

Following collection of the final image, each animal is deeply anesthetized with pentobarbital. Two blood samples are collected on a cardiac puncture using a heparinized syringe followed by a euthanasing dose of saturated potassium chloride solution administered by intercardiac or bolus intravenous injection. The femoral vein containing the thrombus, a similar section of vein of the contralateral (control) leg, sections of the vessel proximal to the thrombus and samples of thigh muscle are then carefully dissected out. The thrombus, coil and coil Dacron fibres are then dissected free of the vessel. The thrombus, saline-washed vessel samples, coil and coil Dacron fibres are separated, and each sample is placed in a pre-weighed test tube. The samples are weighed and counted in a gamma well counter in the Tc-99m channel, along with known fractions of the injected doses.

Fresh thrombus weight, percent injected dose (%ID)/g in the thrombus and blood obtained just prior to euthanasia and thrombus/blood and thrombus/muscle ratios are determined. From the computer-stored images, thrombus/background ratios are determined by analysis of the counts/pixel measured in regions-of-interest (ROI) drawn over the thrombus and adjacent muscle.

## EXAMPLE 5

### Localization and *In Vivo* Imaging of Atherosclerotic Plaque using Tc-99m Labeled Compound P215 in the Hypercholesterol Rabbit Model

New Zealand White (NZW) rabbits of both sexes and weighing 2-3kg were divided into two groups. The control group consisted of 6 rabbits that were housed and fed commercial rabbit chow (Purina). Sixteen rabbits, the HC group, were fed a standardized, cholesterol-rich diet (rabbit chow mixed to a 1% w/w concentration of cholesterol) from seven weeks until 28 weeks of age. All animals were given water *ad libitum.*

Tc-99m labeled P199 (*mercaptoacetyl*GGGRALVDTLKFVTQAEGAK.amide) was prepared as described above. Approximately 1000μg of peptide was labeled with 100-200mCi of Tc-99m and prepared in unit doses of 5-10mCi (12.5-20.0 μg/rabbit; 6-7μg/kg) in 0.5-2mL volume doses. Adult rabbits were dosed with Tc-99m labeled peptide intravenously in a lateral ear vein by slow bolus infusion (approximately 0.1mL/min). A gamma camera fitted with a pinhole collimator (5mm aperture) and energy window set for Tc-99m and programmed to accumulate 500,000 counts or scan for a desired time. Shortly before imaging, animals were anesthetized with a mixture of ketamine and xylazine (5:1, 1mL/kg intramuscularly).

Gamma camera images were collected at 40°-45° just above the heart (left anterior oblique [LAO] view) to delineate the aortic arch and view the descending aorta. Images were acquired at 15 min and 2h after injection. Supplementary anesthesia was injected as needed prior to each image collection.

At 2.5 h (after a 2h scan), animals were sacrificed with an intravenous dose of sodium pentobarbital. Upon necropsy, the aorta was removed and branching vessels dissected free from the aortic valve to the mid-abdominal region. Using a parallel hole collimator, the aorta was imaged *ex corpora.* Next, the aortae were opened longitudinally and stained with Sudan IV, thereby turning atherosclerotic plaque a deep red brick color. Lipid-free and uninjured aortic endothelium retains its normal, glistening white-pink appearance under these conditions.

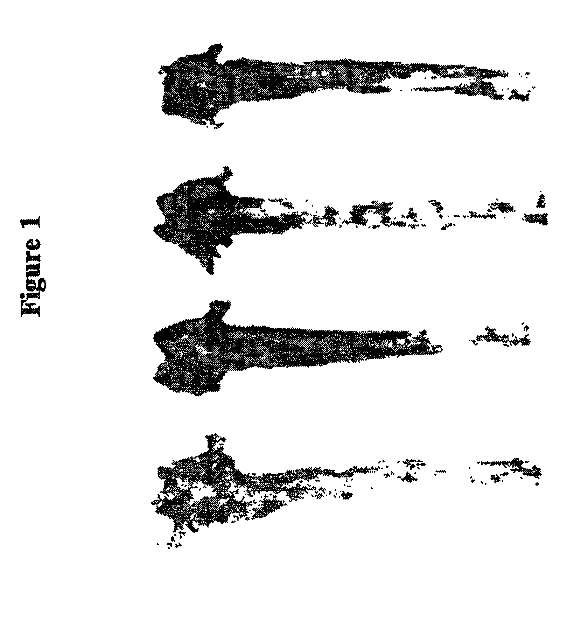

The results of these experiments are shown in Figures 1-3. Both groups of rabbits showed rapid systemic clearance of Tc-99m P199. Control (plaque-free) aortae were only visible for a short time after injection, resulting from circulating, blood-borne radioactivity. Each of the HC-fed NZW rabbit aortae showed a unique pattern and intensity of plaque distribution when imaged *ex corpora.* All the HC aortae had variable amounts of radioactivity accumulation but were consistent in their display of the greatest deposition in the region of the aortic arch, with lesser degrees of accumulation in the distal and proximal segments of the aorta.

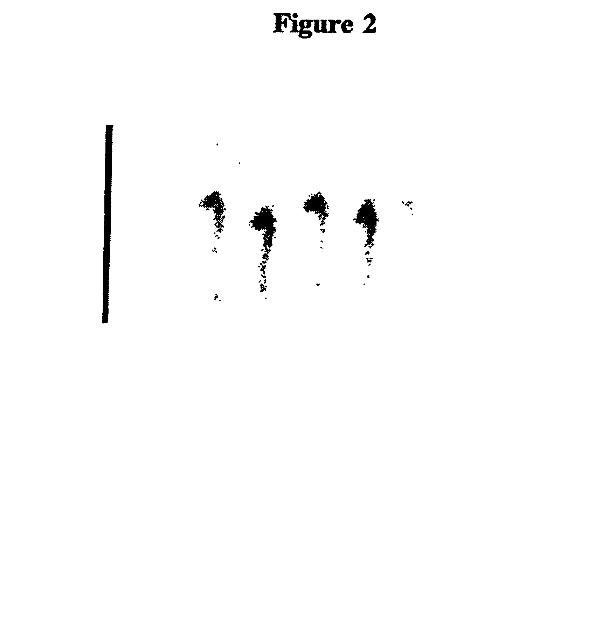

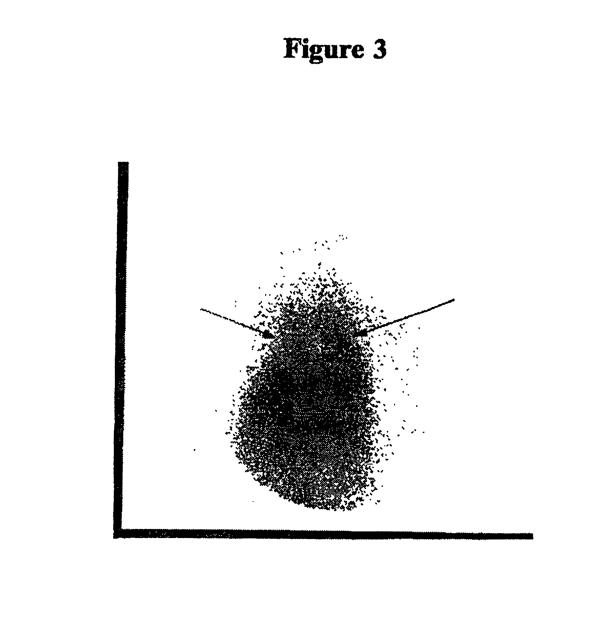

Positive correlations were observed among the *in vivo* and *ex corpora* Tc-99m P199 images and the deposition patterns of Sudan IV in the HC-treated rabbit aortae. In contrast, no control aortae showed any regional uptake of labeled peptide. Figure 1 shows the deposition pattern of Sudan IV in 4 HC-treated and 1 control rabbit aortae. The dark areas indicate the location of atherosclerotic plaque. Figure 2 shows the corresponding *ex corpora* images showing correspondance of radiotracer uptake and plaque visualization. Figure 3 shows the *in vivo* image in an animal of radiotracer uptake and plaque localization in the aortic arch.

These results demonstrate that Tc-99m labeled P199 is capable of imaging atherosclerotic plaque in an animal with high uptake and rapid clearance, facilitating early observation. Additionally, normal aortic tissue shows minimal uptake of labeled P199, thereby reducing the likelihood of artifactual positive scintigraphic images.

## EXAMPLE 6

### Scintigraphic Imaging and Biodistribution of Tc-99m Labeled Peptides in an Animal Model of Infection

New Zealand White (NZW) rabbits of both sexes and weighing 2-3kg are innoculated intramuscularly in the left calf with a potent strain of *Escherichia coli.* After 24 hours, the animals are sedated by intramuscular injection of ketamine and xylazine and then injected with Tc-99m labeled peptide (2-10mCi, $\leq$ 150$\mu$g). The animals are then positioned supine in the filed of view of a gamma cammera (LEAP collimator/photopeaked for Tc-99m) to be imaged. The animals are imaged over the first hour post-injection, and then at approximately 1 hour intervals for the next 3 hours. Animals are allowed to recover between image acquisitions and re-anesthetized as needed.

Upon completion of the final imaging, each animal is sacrificed with an intravenous overdose of sodium pentobarbital, then dissected to obtain samples of blood and of infected and control tissue. Tissue samples are weighed and counted using a gamma radition counter; a standard amount the injected dose is counted in parallel with each sample as a control. From these data the percent of the injected dose per gram of tissue remaining in each tissue sample is determined. Ratios of percent of injected dose per gram of infected tissue *versus* non-infected muscle tissue, and of infected muscle tissue *versus* blood, are then calculated for each peptide to demonstrate specific localization of radiolabeled scintigraphic imaging agents of the invention.

## EXAMPLE 7

### Inhibition of [125I-Tyr11]Somatostatin-14 Binding to AR42J Rat Pancreatic Tumor Cell Membranes

The ability of various somatostatin analogues of the invention to bind to somatostatin receptors *in vitro* was demonstrated in an assay of peptide reagent-mediated inhibition of binding of a radiolabeled somatostatin analogue to somatostatin receptor-containing cell membranes.

The rat pancreatic tumor cell line AR42J expressing the somatostatin receptor was cultured in Dulbecco's modified essential media (DMEM) supplemented with 10% fetal calf serum (FCS). and 8mM glutamine in a humidified 5% $CO_2$ atmosphere at 37°C. Harvested cells were homogenized in cold buffer (50mM Tris-HCl, pH 7.4), and the homogenate was then centrifuged at 39,000g for 10min at 4°C. Pellets were washed once with buffer andd then resuspened in ice-cold 10mM Tris-HCl buffer (pH 7.4). Equal aliquots of this cell membrane preparation were then incubated with [125I-Tyr11]somatostatin-14 (Amersham, Arlington Heights, IL) at a final concentration of 0.5nM at 750,000cpm/mL, specific activity 2000Ci/mmol and either a peptide or peptide-rhenium complex of the invention (at a final concentration ranging from $10^{-11}$ to $10^{-6}$M in 50mM HEPES buffer, pH 7.4, containing 1% bovine serum albumin, 5mM $MgCl_2$, 0.02mg/mL bacitracin, 0.02mg/mL phenylmethyl-sulfonylfluoride and 200,000 IU Trasylol) for 25min at 30°C.

After incubation, this membrane mixture was filtered through a polyuethyleneimine-washed GC/F filter (Whatman Ltd., Maidstone, England) using a filtration manifold, and the residue remaining on the filter was washed three times with 5mL cold HEPES buffer. the filter and a sample of the filter washings were then counted on a gamma counter. To assess non-specific binding, the assay was also performed essentially as described in the presence of 200mn unlabeled somatostatin-14. Data analysis included Hill plots of the data to yield inhibition constants as described by Bylund and Yamamura (1990, *Methods in Neurotransmitter Receptor Analysis*, Yamamura *et al.*, eds., Raven Press: N.Y.). The results obtained using this assay with the reagents of the invention are as follows:

## TABLE III

| Peptide | $K_i(nM)$ |
|---|---|
| *cyclo*(*N*-methyl)FYW$_D$KV.Hcy.(CH$_2$CO.GGCKK.amide) | 0.26 |
| *cyclo*(*N*-methyl)FYW$_D$KV.Hcy.(CH$_2$CO.GGCK.amide) | 2.5 |
| *cyclo*(*N*-methyl)FYW$_D$KV.Hcy.(CH$_2$CO.GGC.amide) | 2.6 |

These results demonstrate that peptide reagents of the inveniton bind with high affinity to somatostatin receptors in *vitro.*

**EXAMPLE 8**

**Localization and *In Vivo* Imaging of Somatostatin Receptor (SSTR)-Expressing Tumors in Rats**

*In vivo* imaging of somatostatin receptors expressed by rat tumor cells was performed essentially as described by Bakker *et al.* (1991, *Life Sciences* 49: 1593-1601).

CA20948 rat pancreatic tumor cells, thawed from frozen harvested tumor brei, were implanted intramuscularly in a suspension of 0.05 to 0.1 mL/animal, into the right hind thigh of 6 week old Lewis rats. The tumors were allowed to grow to approximately 0.5 to 2g, harvested, and tumor brei was used to implant a second, naive set of Lewis rats. Passaging in this fashion was repeated to generate successive generations of tumor-bearing animals. The tumor-bearing animals used for the *in vivo* studies were usually from the third to fifth passage and carried 0.2 to 2g tumors.

For studies of the specificity of radiotracer localization in the tumors, selected animals were given an subcutaneous SSTR-blocking dose (4 mg/kg) of octreotide 30 minutes prior to injection of the radiotracer. (This protocol has been shown by Bakker *et al.* to result in a lowering of '"In-[DTPA]octreotide tumor uptake by 40%.)

Third- to fifth-passage CA20948 tumor-bearing Lewis rats were restrained and injected intravenously *via* the dorsal tail vein with a dose of 0.15-0.20 mCi [99mTc]-labeled peptide (corresponding to 3 to 8 µg peptide in 0.2 to 0.4 mL).

At selected times, the animals were sacrificed by cervical dislocation and selected necropsy was performed. Harvested tissue samples were weighed and counted along with an aliquot of the injected dose in a gamma well-counter.

The 90-minute biodistribution results of selected radiolabeled peptides are presented in Table IV. Notably, [99mTc]-P587, [99mTc]-P617, [99mTc]-P726, and [99mTc]-P736 showed very high tumor uptake and tumor/blood ratios demonstrating their high specific uptake in target (tumor) tissue.

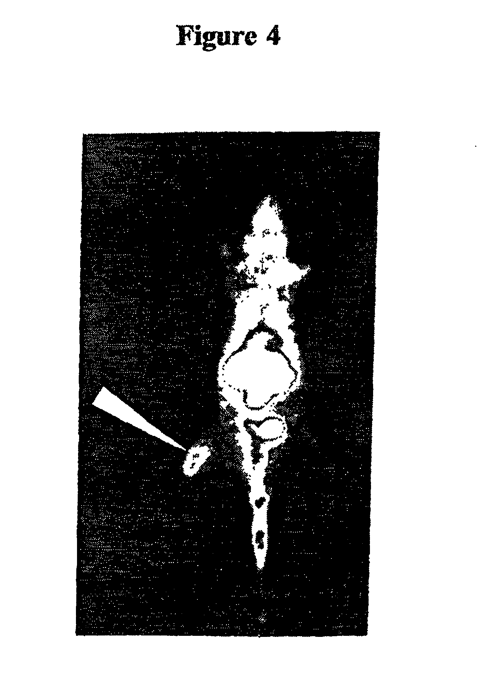

Figure 4 shows an image of [99mTc]-P587 in a tumor-bearing rat. The high uptake in the tumor in the lower leg (arrow) is clearly visible.

It should be understood that the foregoing disclosure emphasizes certain specific embodiments of the invention and that all modifications or alternatives equivalent thereto are within the spirit and scope of the invention as set forth in the appended claims.

## TABLE IV

| No. | Peptides | %ID/g | | |
|-----|----------|-------|---|---|
| | | Tumor | Blood | Tumor/Blood |
| P736 | cyclo(N-methyl)FYW$_D$KV.Hcy.(CH$_2$co.GGCRK.amide) | 2.1 | 0.24 | 9 |
| P587 | cyclo(N-methyl)FYW$_D$KV.Hcy.(CH$_2$co.GGCK.amide) | 3.4 | 0.61 | 6 |
| P617 | cyclo(N-methyl)FYW$_D$KV.Hcy.(CH$_2$co.GGCR.amide) | 6.7 | 0.73 | 9 |
| P726 | cyclo(N-methyl)FYW$_D$KV.Hcy.(CH$_2$co.KKC.amide) | 2.5 | 0.30 | 8 |

## Claims

1. A reagent for preparing a scintigraphic imaging agent for imaging a site within a mammalian body, comprising a specific binding compound that is less than 10,000 daltons in molecular weight covalently linked to a radiolabel complexing moiety having formula:

15

$$R^1\text{-CO-(amino acid)}^1\text{-(amino acid)}^2\text{-Z}$$

wherein (amino acid)$^1$ and (amino acid)$^2$ are each independently any primary α- or β-amino acid that does not comprise a thiol group; Z is a thiol-containing moiety that is cysteine, homocysteine, isocysteine, penicillamine, 2-mercaptoethylamine or 3-mercaptopropylamine;
R$^1$ is lower (C$^1$-C$^4$) alkyl or a covalent linkage to the specific binding compound;

wherein when Z is cysteine, homocysteine, isocysteine or penicillamine, the carbonyl group of said moiety is covalently linked to a NR$^3$R$^4$ group, an amino acid or a peptide comprising 2 to 10 amino acids, wherein R$^3$ and R$^4$ are each independently H or lower (C$^1$-C$^4$) alkyl; or

II.

$$Y\text{-(amino acid)}^2\text{-(amino acid)}^1\text{-NHR}^2$$

wherein Y is a thiol-containing moiety that is cysteine, homocysteine, isocysteine or penicillamine;
(amino acid)$^1$ and (amino acid)$^2$ are each independently any primary α- or β-amino acid that does not comprise a thiol group;
R$^2$ is H or lower (C$^1$-C$^4$) alkyl or a covalent linkage to the specific binding compound;

wherein the amino group of Y is covalently linked to an amino acid or a peptide comprising 2 to 10 amino acids; and
wherein the radiolabel complexing moiety is covalently linked to the specific binding compound through R$^1$, R$^2$, a sidechain group of the sidechain of (amino acid)$^1$ or (amino acid)$^2$, or the amino or carboxyl group of cysteine, homocysteine, isocysteine or penicillamine.

2. A reagent of claim 1 wherein the radiolabel complexing moiety is selected from moieties having the formula:

$$\text{-(amino acid)}^1\text{-(amino acid)}^2\text{-(amino thiol).}$$

and (mercaptocarboxylic acid)-(amino acid)$^1$-(amino acid)$^2$-.
wherein (amino acid)$^1$ and (amino acid)$^2$ are each independently any primary α- or β-amino acid;
(amino thiol) is selected from the group consisting of cysteine, isocysteine, homocysteine, penicillamine, 2-mercaptoethylamine, and 3-mercaptopropylamine; and
(mercaptocarboxylic acid) is selected from the group consisting of cysteine, isocysteine, homocysteine or penicillamine; or

wherein the radiolabel complexing moiety is Gly-Gly-Cys- or Cys-Gly-Gly.

3. A composition of matter comprising a reagent according to claim 1 selected from:

cyclo(*N*-methyl)FYW$_D$KV.Hcy.(CH$_2$CO.GGC.amide)
cyclo(*N*-methyl)FYW$_D$KV.Hcy.(CH$_2$CO.GGCK.amide)
cyclo(*N*-methyl)FYW$_D$KV.Hcy.(CH$_2$CO.GGCR.amide)
cyclo(*N*-methyl)FYW$_D$KV.Hcy.(CH$_2$CO.GGCRD.amide)
cyclo(*N*-methyl)FYW$_D$KV.Hcy.(CH$_2$CO.GGCRK.amide)
cyclo(*N*-methyl)FYW$_D$KV.Hcy.(CH$_2$CO.GGCRR.amide)
cyclo(*N*-methyl)FYW$_D$KV.Hcy.(CH$_2$CO.GGCKK.amide)
cyclo(*N*-methyl)FYW$_D$KV.Hcy.(CH$_2$CO.GGCKKK.amide)
cyclo(*N*-methyl)FYW$_D$KV.Hcy.(CH$_2$CO.GGC.Orn.amide)
cyclo(*N*-methyl)FYW$_D$KV.Hcy.(CH$_2$CO.GGCKDK.amide)
cyclo(*N*-methyl)FYW$_D$KV.Hcy.(CH$_2$CO.GGC.Orn.D.Orn.amide)
cyclo(*N*-methyl)FYW$_D$KV.Hcy.(CH$_2$CO.GGC.Orn.D.amide)
cyclo(*N*-methyl)FYW$_D$KV.Hcy.(CH$_2$CO.KKC.amide)
cyclo(*N*-methyl)FYW$_D$KV.Hcy.(CH$_2$CO.KRC.amide)
cyclo(*N*-methyl)FYW$_D$KV.Hcy.(CH$_2$CO.RRC.amide)

cyclo(*N*-methyl)FYW_DKV.Hcy.(CH_2CO.KKCK.amide)
cyclo(*N*-methyl)FYW_DKV.Hcy.(CH_2CO.GRCK.amide)
cyclo(*N*-methyl)FYW_DKV.Hcy.(CH_2CO.GKCR.amide)
CH_2CO.Y_D.Apc.GDCGGC_{Acm}GC_{Acm}GGCG.amide
CH_2CO.Y_D.Apc.GDCGGSSGGCG.amide
CH_2CO.Y_D.Apc.GDCGGCG.amide
or CH_2CO.Y_D.Amp.GDCKGCG.amide

4. A reagent according to claim 1 or claim 2
   wherein the specific binding compound is a specific binding peptide comprising 4 to 100 amino acids and/or is comprised of linear or cyclic peptides.

5. A reagent of any one of claims 1, 2 or 4
   wherein the specific binding peptide and radiolabel binding moiety are covalently linked through one or more amino acids.

6. A reagent of any one of claims 1, 2 or 4
   wherein the reagent further comprises a polyvalent linking moiety covalently linked to a multiplicity of specific binding compounds and also covalently linked to a multiplicity of radiolabel-complexing moieties to comprise a reagent for preparing a multimeric polyvalent scintigraphic imaging agent, wherein the molecular weight of the multimeric polyvalent scintigraphic imaging agent is less than about 20,000 daltons; optionally wherein the polyvalent linking moiety is bis-succinimdylmethylether, 4-(2,2-dimethylacetyl)benzoic acid, *tris*(succinimidylethyl) amine, and 1,2-*bis*-[2-(chloroacetamido)ethoxy]ethane or a derivative thereof.

7. A scintigraphic imaging agent comprising a reagent according to any one of claims 1 to 6 wherein the radiolabel binding moiety is bound to a radiolabel, e.g. technetium-99m.

8. A complex formed by reacting a reagent of any one of claims 1 to 6 with technetium-99m in the presence of a reducing agent or by labeling said reagent with technetium-99m by ligand exchange of a prereduced technetium-99m complex; optionally wherein the reducing agent is selected from dithionite ion, stannous ion or ferrous ion.

9. A kit for preparing a radiopharmaceutical preparation, said kit comprising a sealed vial containing a predetermined quantity of a reagent of any one of claims 1 to 6 and a sufficient amount of reducing agent to label the reagent with technetium-99m.

10. A method for labeling a reagent according to any one of claims 1 to 6 comprising reacting the reagent with technetium-99m in the presence of a reducing agent, optionally wherein the reducing agent is selected from dithionite ion, stannous ion or ferrous ion.

11. A composition of matter having formula:

    cyclo(*N*-methyl)FYW_DKV.Hcy.(CH_2CO.GGC.amide)
    cyclo(*N*-methyl)FYW_DKV.Hcy.(CH_2CO.GGCK.amide)
    cyclo(*N*-methyl)FYW_DKV.Hcy.(CH_2CO.GGCR.amide)
    cyclo(*N*-methyl)FYW_DKV.Hcy.(CH_2CO.GGCRD.amide)
    cyclo(*N*-methyl)FYW_DKV.Hcy.(CH_2CO.GGCRK.amide)
    cyclo(*N*-methyl)FYW_DKV.Hcy.(CH_2CO.GGCRR.amide)
    cyclo(*N*-methyl)FYW_DKV.Hcy.(CH_2CO.GGCKK.amide)
    cyclo(*N*-methyl)FYW_DKV.Hcy.(CH_2CO.GGCKKK.amide)
    cyclo(*N*-methyl)FYW_DKV.Hcy.(CH_2CO.GGC.Orn.amide)
    cyclo(*N*-methyl)FYW_DKV.Hcy.(CH_2CO.GGCKDK.amide)
    cyclo(*N*-methyl)FYW_DKV.Hcy.(CH_2CO.GGC.Orn.D.Orn.amide)
    cyclo(*N*-methyl)FYW_DKV.Hcy.(CH_2CO.GGC.Orn.D.amide)
    cyclo(*N*-methyl)FYW_DKV.Hcy.(CH_2CO.KKC.amide)
    cyclo(*N*-methyl)FYW_DKV.Hcy.(CH_2CO.KRC.amide)
    cyclo(*N*-methyl)FYW_DKV.Hcy.(CH_2CO.RRC.amide)
    cyclo(*N*-methyl)FYW_DKV.Hcy.(CH_2CO.KKCK.amide)
    cyclo(*N*-methyl)FYW_DKV.Hcy.(CH_2CO.GRCK.amide)

*cyclo*(*N*-methyl)FYW$_D$KV.Hcy.(CH$_2$CO.GKCR.amide)
CH$_2$CO.Y$_D$.Apc.GDCGGC$_{Acm}$GC$_{Acm}$GGCG.amide
CH$_2$CO.Y$_D$.Apc.GDCGGSSGGCG.amide
CH$_2$CO.Y$_D$.Apc.GDCGGCG.amide
or CH$_2$CO.Y$_D$.Amp.GDCKGCG.amide.

**12.** A composition of matter according to claim 11 that is radiolabeled with technetium-99m.

**13.** A reagent of any one of claims 1 to 6, 11 or 12, an agent of claim 7, or a complex of claim 8 for use as a pharmaceutical.

**14.** The use of a reagent of any one of claims 1 to 6, 11 or 12, an agent of claim 7, or a complex of claim 8 in the manufacture of a medicament for imaging a site within a mammalian body, optionally wherein the imaged site is a thrombus site or a site of infection.

**15.** An article of manufacture comprising a sealed vial containing a predetermined quantity of the composition of matter of claim 11 and a sufficient amount of reducing agent to label the composition with technetium-99m.

**Patentansprüche**

**1.** Reagens zur Herstellung eines Mittels zur szintigraphischen Bilddarstellung zur Abbildung einer Stelle im Körper eines Säugetieres, enthaltend eine spezifisch bindende Verbindung mit einem Molekulargewicht von weniger als 10 000 Dalton, die kovalent an eine einen radioaktiven Marker komplexierende Einheit der Formel

I.

R$^1$-CO-(Aminosäure)$^1$-(Aminosäure)$^2$-Z,

wobei (Aminosäure)$^1$ und (Aminosäure)$^2$ jeweils unabhängig voneinander für eine beliebige primäre $\alpha$- oder $\beta$-Aminosäure, die keine Thiolgruppe enthält, stehen;
Z für eine thiolhaltige Einheit steht, bei der es sich um Cystein, Homocystein, Isocystein, Penicillamin, 2-Mercaptoethylamin oder 3-Mercaptopropylamin handelt;
R$^1$ für niederes (C$_1$-C$_4$)-Alkyl oder eine kovalente Bindung an die spezifisch bindende Verbindung steht;
wobei, wenn Z für Cystein, Homocystein, Isocystein oder Penicillamin steht, die Carbonylgruppe der Einheit kovalent an eine NR$^3$R$^4$-Gruppe, eine Aminosäure oder ein Peptid mit 2 bis 10 Aminosäuren gebunden ist, wobei R$^3$ und R$^4$ jeweils unabhängig voneinander für H oder niederes (C$_1$-C$_4$)-Alkyl stehen; oder

II.

Y-(Aminosäure)$^2$-(Aminosäure)$^1$-NHR$^2$

wobei Y für eine thiolhaltige Einheit steht, bei der es sich um Cystein, Homocystein, Isocystein oder Penicillamin handelt;
(Aminosäure)$^1$ und (Aminosäure)$^2$ jeweils unabhängig voneinander für eine beliebige primäre $\alpha$- oder $\beta$-Aminosäure, die keine Thiolgruppe enthält, stehen;
R$^2$ für H oder niederes (C$_1$-C$_4$)-Alkyl oder eine kovalente Bindung an die spezifisch bindende Verbindung steht;
wobei die Aminogruppe von Y kovalent an eine Aminosäure oder ein Peptid mit 2 is 10 Aminosäuren gebunden ist; und
wobei die den radioaktiven Marker komplexierende Einheit über R$^1$, R$^2$, eine Seitenkettengruppe der Seitenkette von (Aminosäure)$^1$ oder (Aminosäure)$^2$ oder die Amino- oder Carboxylgruppe von Cystein, Homocystein, Isocystein oder Penicillamin kovalent an die spezifisch bindende Verbindung gebunden ist,
gebunden ist.

**2.** Reagens nach Anspruch 1, wobei die den radioaktiven Marker komplexierende Einheit aus Einheiten der folgenden Formeln ausgewählt ist:

-(Aminosäure)$^1$-(Aminosäure)$^2$-(Aminothiol),

und (Mercaptocarbonsäure)-(Aminosäure)$^1$-(Aminosäure)$^2$,

wobei (Aminosäure)$^1$ und (Aminosäure)$^2$ jeweils unabhängig voneinander für eine beliebige primäre α- oder β-Aminosäure stehen;

(Aminothiol) aus der aus Cystein, Isocystein, Homocystein, Penicillamin, 2-Mercaptoethylamin und 3-Mercaptopropylamin bestehenden Gruppe ausgewählt ist; und

(Mercaptocarbonsäure) aus der aus Cystein, Isocystein, Homocystein und Penicillamin bestehenden Gruppe ausgewählt ist; oder

wobei es sich bei der den radioaktiven Marker komplexierenden Einheit um Gly-Gly-Cys- oder Cys-Gly-Gly handelt.

3. Stoffzusammensetzung, enthaltend ein Reagens nach Anspruch 1 ausgewählt aus:

*Cyclo*(*N*-methyl)FYW$_D$KV.Hcy. (CH$_2$CO.GGC.Amid)
*Cyclo*(*N*-methyl)FYW$_D$KV.Hcy. (CH$_2$CO.GGCK.Amid)
*Cyclo*(*N*-methyl)FYW$_D$KV.Hcy. (CH$_2$CO.GGCR.Amid)
*Cyclo*(*N*-methyl)FYW$_D$KV.Hcy. (CH$_2$CO.GGCRD.Amid)
*Cyclo*(*N*-methyl)FYW$_D$KV.Hcy. (CH$_2$CO.GGCRK.Amid)
*Cyclo*(*N*-methyl)FYW$_D$KV.Hcy. (CH$_2$CO.GGCRR.Amid)
*Cyclo*(*N*-methyl)FYW$_D$KV.Hcy. (CH$_2$CO.GGCKK.Amid)
*Cyclo*(*N*-methyl)FYW$_D$KV.Hcy. (CH$_2$CO.GGCKKK.Amid)
*Cyclo*(*N*-methyl)FYW$_D$KV.Hcy. (CH$_2$CO.GGC.Orn.Amid)
*Cyclo*(*N*-methyl)FYW$_D$KV.Hcy. (CH$_2$CO.GGCKDK.Amid)
*Cyclo*(*N*-methyl)FYW$_D$KV.Hcy. (CH$_2$CO.GGC.Orn.D.Orn.Amid)
*Cyclo*(*N*-methyl)FYW$_D$KV.Hcy. (CH$_2$CO.GGC.Orn.D.Amid)
*Cyclo*(*N*-methyl)FYW$_D$KV.Hcy. (CH$_2$CO.KKC.Amid)
*Cyclo*(*N*-methyl)FYW$_D$KV.Hcy. (CH$_2$CO.KRC.Amid)
*Cyclo*(*N*-methyl)FYW$_D$KV.Hcy. (CH$_2$CO.RRC.Amid)
*Cyclo*(*N*-methyl)FYW$_D$KV.Hcy. (CH$_2$CO.KKCK.Amid)
*Cyclo*(*N*-methyl)FYW$_D$KV.Hcy. (CH$_2$CO.GRCK.Amid)
*Cyclo*(*N*-methyl)FYW$_D$KV.Hcy. (CH$_2$CO.GKCR.Amid)
CH$_2$CO.Y$_D$.Apc.GDCGGC$_{Acm}$GC$_{Acm}$GGCG.Amid
CH$_2$CO.Y$_D$.Apc.GDCGGSSGGCG.Amid
CH$_2$CO.Y$_D$.Apc.GDCGGCG.Amid
oder CH$_2$CO.Y$_D$.Amp.GDCKGCG.Amid.

4. Reagens nach Anspruch 1 oder Anspruch 2, wobei es sich bei der spezifisch bindenden Verbindung um ein spezifisch bindendes Peptid, das 4 bis 100 Aminosäuren und/oder lineare oder cyclische Peptide enthält, handelt.

5. Reagens nach einem der Ansprüche 1, 2 oder 4, wobei das spezifisch bindende Peptid und die den radioaktiven Marker bindende Einheit kovalent über eine oder mehrere Aminosäuren verbunden sind.

6. Reagens nach einem der Ansprüche 1, 2 oder 4, wobei das Reagens weiterhin eine polyvalente Verbindungseinheit umfaßt, die kovalent an eine Vielzahl spezifisch bindender Verbindungen gebunden ist und weiterhin kovalent an eine Vielzahl von radioaktive Marker bindende Einheiten gebunden ist, so daß es ein Reagens zur Herstellung eines multimeren, polyvalenten Mittels zur szintigraphischen Bilddarstellung enthält, wobei das Molekulargewicht des multimeren, polyvalenten Mittels zur szintigraphischen Bilddarstellung weniger als etwa 20 000 Dalton beträgt; wobei es sich bei der mehrwertigen Verbindungseinheit gegebenenfalls um *Bis*succinimidylmethylether, 4-(2,2-Dimethylacetyl)-benzoesäure, *Tris*(succinimidylethyl)amin und 1,2-*Bis*-[2-(chloracetamido)ethoxy]ethan oder ein Derivat davon handelt.

7. Mittel zur szintigraphischen Bilddarstellung, enthaltend ein Reagens nach einem der Ansprüche 1 bis 6, wobei die den radioaktiven Marker bindende Einheit an einen Radiomarker, z.B. Technetium-99m, gebunden ist.

8. Komplex, gebildet durch Umsetzung eines Reagens nach einem der Ansprüche 1 bis 6 mit Technetium-99m in Gegenwart eines Reduktionsmittels oder durch Markieren des Reagens mit Technetium-99m durch Ligandenaustausch mit einem vorreduzierten Technetium-99m-Komplex; wobei das Reduktionsmittel gegebenenfalls aus Dithionitionen, Zinn(II)-Ionen oder Eisen(II)-Ionen ausgewählt ist.

9. Kit zur Herstellung einer radiopharmazeutischen Zubereitung, wobei das Kit aus einem verschlossenen Vial besteht, das eine vorbestimmte Menge eines Reagens nach einem der Ansprüche 1 bis 6 und ein Reduktionsmittel in einer Menge enthält, die ausreicht, um das Reagens mit Technetium-99m zu markieren.

10. Verfahren zur Markierung eines Reagens nach einem der Ansprüche 1 bis 6, bei dem man das Reagens in Gegenwart eines Reduktionsmittels mit Technetium-99m umsetzt, wobei das Reduktionsmittel gegebenenfalls aus Dithionitionen, Zinn(II)-Ionen oder Eisen(II)-Ionen ausgewählt ist.

11. Stoffzusammensetzung der Formel:

*Cyclo*(*N*-methyl)FYW$_D$KV.Hcy. (CH$_2$CO.GGC.Amid)
*Cyclo*(*N*-methyl) FYW$_D$KV.Hcy. (CH$_2$CO.GGCK.Amid)
*Cyclo*(*N*-methyl)FYW$_D$KV.Hcy. (CH$_2$CO.GGCR.Amid)
*Cyclo*(*N*-methyl)FYW$_D$KV.Hcy. (CH$_2$CO.GGCRD.Amid)
*Cyclo*(*N*-methyl)FYW$_D$KV.Hcy. (CH$_2$CO.GGCRK.Amid)
*Cyclo*(*N*-methyl)FYW$_D$KV.Hcy. (CH$_2$CO.GGCRR.Amid)
*Cyclo*(*N*-methyl)FYW$_D$KV.Hcy. (CH$_2$CO.GGCKK.Amid)
*Cyclo*(*N*-methyl)FYW$_D$KV.Hcy. (CH$_2$CO.GGCKKK.Amid)
*Cyclo*(*N*-methyl)FYW$_D$KV.Hcy. (CH$_2$CO.GGC.Orn.Amid)
*Cyclo*(*N*-methyl)FYW$_D$KV.Hcy. (CH$_2$CO.GGCKDK.Amid)
*Cyclo*(*N*-methyl)FYW$_D$KV.Hcy. (CH$_2$CO.GGC.Orn.D.Orn.Amid)
*Cyclo*(*N*-methyl)FYW$_D$KV.Hcy. (CH$_2$CO.GGC.Orn.D.Amid)
*Cyclo*(*N*-methyl)FYW$_D$KV.Hcy. (CH$_2$CO.KKC.Amid)
*Cyclo*(*N*-methyl)FYW$_D$KV.Hcy. (CH$_2$CO.KRC.Amid)
*Cyclo*(*N*-methyl)FYW$_D$KV.Hcy. (CH$_2$CO.RRC.Amid)
*Cyclo*(*N*-methyl)FYW$_D$KV.Hcy. (CH$_2$CO.KKCK.Amid)
*Cyclo*(*N*-methyl)FYW$_D$KV.Hcy. (CH$_2$CO.GRCK.Amid)
*Cyclo*(*N*-methyl)FYW$_D$KV.Hcy. (CH$_2$CO.GKCR.Amid)
CH$_2$CO.Y$_D$.Apc.GDCGGC$_{Acm}$GC$_{Acm}$GGCG.Amid
CH$_2$CO.Y$_D$.Apc.GDCGGSSGGCG.Amid
CH$_2$CO.Y$_D$.Apc.GDCGGCG.Amid
oder CH$_2$CO.Y$_D$.Amp.GDCKGCG.Amid.

12. Stoffzusammensetzung nach Anspruch 11, die mit Technetium-99m radioaktiv markiert ist.

13. Reagens nach einem der Ansprüche 1 bis 6, 11 oder 12, Mittel nach Anspruch 7, oder Komplex nach Anspruch 8 zur Verwendung als Pharmazeutikum.

14. Verwendung eines Reagens nach einem der Ansprüche 1 bis 6, 11 oder 12, eines Mittels nach Anspruch 7 oder eines Komplexes nach Anspruch 8, zur Herstellung eines Medikaments zur Abbildung einer Stelle im Körper eines Säugetiers, wobei es sich bei der abgebildeten Stelle gegebenenfalls um eine Thrombusstelle oder eine Infektionsstelle handelt.

15. Hergestelltes Produkt, enthaltend ein verschlossenes Vial mit einer vorbestimmten Menge der Stoffzusammensetzung nach Anspruch 11 und ein Reduktionsmittel in einer Menge, die ausreicht, um die Zusammensetzung mit Technetium-99m zu markieren.

**Revendications**

1. Réactif pour la préparation d'un agent d'imagerie scintigraphique pour obtenir l'image d'un site dans un corps de mammifère, comprenant un composé de liaison spécifique qui est d'un poids moléculaire inférieur à 10 000 daltons lié de manière covalente à une partie complexant un radiomarqueur présentant la formule :

I.

$$R^1\text{-CO-(acide aminé)}^1\text{-(acide aminé)}^2\text{-Z}$$

dans laquelle  (acide aminé)$^1$ et (acide aminé)$^2$ sont chacun indépendamment un quelconque acide aminé primaire α ou β qui ne comprend pas un groupement thiol ; Z est une partie contenant un thiol qui est une cystéine, une homocystéine, une isocystéine, une pénicillamine, une 2-mercaptoéthylamine ou une 3-mercaptopropylamine ;
R$^1$ est un alkyle inférieur en C$_1$-C$_4$ ou une liaison covalente au composé de liaison spécifique ;

dans lequel lorsque Z est une cystéine, une homocystéine, une isocystéine ou une pénicillamine, le groupement carbonyle de ladite partie est lié de manière covalente à un groupement NR$^3$R$^4$, un acide aminé ou un peptide comprenant 2 à 10 acides aminés, dans laquelle R$^3$ et R$^4$ sont chacun indépendamment un quelconque H ou un alkyle inférieur en C$_1$-C$_4$ ; ou

II.

$$Y\text{-(acide aminé)}^2\text{-(acide aminé)}^1\text{-NHR}^2$$

dans laquelle  Y est une partie contenant un thiol qui est une cystéine, une homocystéine, une isocystéine ou une pénicillamine ;
(acide aminé)$^1$ et (acide aminé)$^2$ sont chacun indépendamment un quelconque acide aminé primaire α ou β qui ne comprend pas un groupement thiol ; R$^2$ est H ou un alkyle inférieur en C$_1$-C$_4$ ou une liaison covalente au composé de liaison spécifique ;

dans lequel le groupement aminé de Y est lié de manière covalente à un acide aminé ou à un peptide comprenant 2 à 10 acides aminés ; et
dans lequel la partie complexant un radiomarqueur est liée de manière covalente au composé de liaison spécifique par R$^1$, R$^2$, un groupement de la chaîne latérale de l'(acide aminé)$^1$ ou de l'(acid aminé)$^2$, ou le groupement aminé ou carboxyle de la cystéine, homocystéine, isocystéine ou pénicillamine.

2.  Réactif suivant la revendication 1, dans lequel la partie complexant un radiomarqueur est sélectionnée parmi des parties présentant la formule :

- (acide aminé)$^1$-(acide aminé)$^2$-(thiol aminé),

et  (acide mercaptocarboxylique)-(acide aminé)$^1$-(acide aminé)$^2$-,

dans lesquelles  (acide aminé)$^1$ et (acide aminé)$^2$ sont chacun indépendamment un quelconque acide aminé primaire α ou β ;
(thiol aminé) est sélectionné parmi le groupe existant d'une cystéine, d'une isocystéine, d'une homocystéine, d'une pénicillamine, d'une 2-mercaptoéthylamine et d'une 3-mercaptopropylamine ; et
(acide mercaptocarboxylique) est sélectionné parmi le groupe existant d'une cystéine, d'une isocystéine, d'une homocystéine ou d'une pénicillamine ; ou

dans lequel la partie complexant un radiomarqueur est Gly-Gly-Cys- ou Cys-Gly-Gly.

3.  Composition de matière comprenant un réactif suivant la revendication 1 sélectionné parmi :

cyclo(*N*-méthyl)FYW$_D$KV.Hcy. (CH$_2$CO.GGC.amide)
cyclo(*N*-méthyl)FYW$_D$KV.Hcy. (CH$_2$CO.GGCK.amide)
cyclo(*N*-méthyl)FYW$_D$KV.Hcy. (CH$_2$CO.GGCR.amide)
cyclo(*N*-méthyl)FYW$_D$KV.Hcy. (CH$_2$CO.GGCRD.amide)
cyclo(*N*-méthyl)FYW$_D$KV.Hcy. (CH$_2$CO.GGCRK.amide)

*cyclo*(*N*-méthyl)FYW$_D$KV.Hcy. (CH$_2$CO.GGCRR.amide)
*cyclo*(*N*-méthyl)FYW$_D$KV.Hcy. (CH$_2$CO.GGCKK.amide)
*cyclo*(*N*-méthyl)FYW$_D$KV.Hcy. (CH$_2$CO.GGCKKK.amide)
*cyclo*(*N*-méthyl)FYW$_D$KV.Hcy. (CH$_2$CO.GGC.Orn.amide)
*cyclo*(*N*-méthyl)FYW$_D$KV.Hcy. (CH$_2$CO.GGCKDK.amide)
*cyclo*(*N*-méthyl)FYW$_D$KV.Hcy. (CH$_2$CO.GGC.Orn.D.Orn.amide)
*cyclo*(*N*-méthyl)FYW$_D$KV.Hcy. (CH$_2$CO.GGC.Orn.D.amide)
*cyclo*(*N*-méthyl)FYW$_D$KV.Hcy. (CH$_2$CO.KKC.amide)
*cyclo*(*N*-méthyl)FYW$_D$KV.Hcy. (CH$_2$CO.KRC.amide)
*cyclo*(*N*-méthyl)FYW$_D$KV.Hcy. (CH$_2$CO.RRC.amide)
*cyclo*(*N*-méthyl)FYW$_D$KV.Hcy. (CH$_2$CO.KKCK.amide)
*cyclo*(*N*-méthyl)FYW$_D$KV.Hcy. (CH$_2$CO.GRCK.amide)
*cyclo*(*N*-méthyl)FYW$_D$KV.Hcy. (CH$_2$CO.GKCR.amide)
CH$_2$CO.Y$_D$.Apc.GDCGGC$_{Acm}$GC$_{Acm}$GGCG.amide
CH$_2$CO.Y$_D$.Apc.GDCGGSSGGCG.amide
CH$_2$CO.Y$_D$.Apc.GDCGGCG.amide
ou CH$_2$CO.Y$_D$.Amp.GDCKGCG.amide.

**4.** Réactif suivant la revendication 1 ou la revendication 2, dans lequel le composé de liaison spécifique est un peptide de liaison spécifique comprenant 4 à 100 acides aminés et/ou comprend des peptides linéaires ou cycliques.

**5.** Réactif suivant l'une quelconque des revendications 1, 2 ou 4, dans lequel le peptide de liaison spécifique et une partie de liaison de radiomarqueur sont liés de manière covalente par un ou plusieurs acides aminés.

**6.** Réactif suivant l'une quelconque des revendications 1, 2 ou 4, dans lequel le réactif comprend en outre une partie polyvalente de liaison liée de manière covalente à une multiplicité de composés de liaison spécifique et également liée de manière covalente à une multiplicité de parties complexant un radiomarqueur pour constituer un réactif de préparation d'un agent multimère polyvalent d'imagerie scintigraphique, dans lequel le poids moléculaire de l'agent multimère polyvalent d'imagerie scintigraphique est inférieur à environ 20 000 daltons ; dans lequel la partie de liaison polyvalente est facultativement du bis-succinimidylméthyléther, de l'acide 4-(2,2-diméthylacétyl)benzoïque, de la tris(succinimidyléthyl)amine, et du 1,2-*bis*-[2-(chloro-acétamido)éthoxy]éthane ou un dérivé de ceux-ci.

**7.** Agent d'imagerie scintigraphique comprenant un réactif suivant l'une quelconque des revendications 1 à 6, dans lequel la partie de liaison de radiomarqueur est liée à un radiomarqueur, par exemple du technétium-99m.

**8.** Complexe formé par la réaction d'un réactif suivant l'une quelconque des revendications 1 à 6, avec du technétium-99m en présence d'un agent réducteur ou par le marquage dudit réactif avec du technétium-99m par échange de ligand d'un complexe de technétium-99m préréduit ; dans lequel l'agent réducteur est facultativement sélectionné parmi l'ion dithionite, l'ion stanneux, ou l'ion ferreux.

**9.** Kit pour la préparation d'une préparation radiopharmaceutique, ledit kit comprenant un flacon scellé contenant une quantité prédéterminée d'un réactif suivant l'une quelconque des revendications 1 à 6 et une quantité suffisante d'agent réducteur pour marquer le réactif avec du technétium-99m.

**10.** Procédé de marquage d'un réactif suivant l'une quelconque des revendications 1 à 6 comprenant la réaction du réactif avec du technétium-99m en présence d'un agent réducteur, dans lequel l'agent réducteur est facultativement sélectionné parmi l'ion dithionite, l'ion stanneux ou l'ion ferreux.

**11.** Composition de matière présentant la formule :

*cyclo*(*N*-méthyl)FYW$_D$KV.Hcy. (CH$_2$CO.GGC.amide)
*cyclo*(*N*-méthyl)FYW$_D$KV.Hcy. (CH$_2$CO.GGCK.amide)
*cyclo*(*N*-méthyl)FYW$_D$KV.Hcy. (CH$_2$CO.GGCR.amide)
*cyclo*(*N*-méthyl)FYW$_D$KV.Hcy. (CH$_2$CO.GGCRD.amide)
*cyclo*(*N*-méthyl)FYW$_D$KV.Hcy. (CH$_2$CO.GGCRK.amide)
*cyclo*(*N*-méthyl)FYW$_D$KV.Hcy. (CH$_2$CO.GGCRR.amide)
*cyclo*(*N*-méthyl)FYW$_D$KV.Hcy. (CH$_2$CO.GGCKK.amide)
*cyclo*(*N*-méthyl)FYW$_D$KV.Hcy. (CH$_2$CO.GGCKKK.amide)

*cyclo*(*N*-méthyl)FYW$_D$KV.Hcy. (CH$_2$CO.GGC.Orn.amide)
*cyclo*(*N*-méthyl)FYW$_D$KV.Hcy. (CH$_2$CO.GGCKDK.amide)
*cyclo*(*N*-méthyl)FYW$_D$KV.Hcy. (CH$_2$CO.GGC.Orn.D.Orn.amide)
*cyclo*(*N*-méthyl)FYW$_D$KV.Hcy. (CH$_2$CO.GGC.Orn.D.amide)
*cyclo*(*N*-méthyl)FYW$_D$KV.Hcy. (CH$_2$CO.KKC.amide)
*cyclo*(*N*-méthyl)FYW$_D$KV.Hcy. (CH$_2$CO.KRC.amide)
*cyclo*(*N*-méthyl)FYW$_D$KV.Hcy. (CH$_2$CO.RRC.amide)
*cyclo*(*N*-méthyl)FYW$_D$KV.Hcy. (CH$_2$CO.KKCK.amide)
*cyclo*(*N*-méthyl)FYW$_D$KV.Hcy. (CH$_2$CO.GRCK.amide)
*cyclo*(*N*-méthyl)FYW$_D$KV.Hcy. (CH$_2$CO.GKCR.amide)
CH$_2$CO.Y$_D$.Apc.GDCGGC$_{Acm}$GC$_{Acm}$GGCG.amide
CH$_2$CO.Y$_D$.Apc.GDCGGSSGGCG.amide
CH$_2$CO.Y$_D$.Apc.GDCGGCG.amide
ou CH$_2$CO.Y$_D$.Amp.GDCKGCG.amide.

**12.** Composition de matière suivant la revendication 11, qui est radiomarquée avec du technétium-99m.

**13.** Réactif suivant l'une quelconque des revendications 1 à 6, 11 ou 12, agent suivant la revendication 7, ou complexe suivant la revendication 8 pour une utilisation comme produit pharmaceutique.

**14.** Utilisation d'un réactif suivant l'une quelconque des revendications 1 à 6, 11 ou 12, d'un agent suivant la revendication 7, ou d'un complexe suivant la revendication 8 dans la fabrication d'un médicament pour obtenir l'image d'un site dans un corps de mammifère, facultativement dans lequel le site dont on obtient une image est un site de thrombus ou un site d'infection.

**15.** Article de fabrication comprenant un flacon scellé contenant une quantité prédéterminée de la composition de matière suivant la revendication 11 et une quantité suffisante d'agent réducteur pour marquer la composition avec du technétium-99m.

Figure 1

# Figure 2

# Figure 3

# Figure 4